# EUROPEAN PATENT SPECIFICATION

(11) **EP 1 715 919 B1**
(45) Date of publication and mention of the grant of the patent: **23.09.2009**
(21) Application number: 04775193.8
(22) Date of filing: 21.06.2004
(51) Int. Cl.: A61N 5/06

(54) **MEDICAL APPARATUS WITH LIGHT FLUX FOR BIOLOGICAL TREATMENTS**
MEDIZINISCHES GERÄT MIT LICHTFLUSS FÜR BIOLOGISCHE BEHANDLUNGEN
APPAREIL MEDICAL A FLUX LUMINEUX POUR TRAITEMENTS BIOLOGIQUES

(30) Priority: 27.01.2004 RO 200400069
(43) Date of publication of application: 02.11.2006
(73) Proprietor: Degeratu, D. Ion Cristian, Chiajna R-jud. Ilfov (RO); Constantinescu, V. Vladimir, 3rd District, Bucharest (RO)
(72) Inventor: Degeratu, D. Ion Cristian, Chiajna R-jud. Ilfov (RO); Constantinescu, V. Vladimir, 3rd District, Bucharest (RO)
(74) Representative: Tuluca, F. Doina
(86) International application number: PCT/RO2004/000010
(87) International publication number: WO 2005/070500

(56) References cited:
- WO-A-92/13597
- WO-A-03/003903
- WO-A-03/015868

## Description

The invention refers to a medical apparatus that uses the effects of the electromagnetic field on living organisms, using an artificial flux of well-determined color for obtaining some modifications at atomic level that can be used to cure or meliorate some diseases or to relax and refresh the body.

The apparatus is designed for curing energetic-degenerative diseases specific to our living in nowadays society, vices, stress, feeding, sedentary living, heredity or genetic deficiency.

About 100 years ago, the Danish doctor Niels Ryberg Finsen laid the foundation of modem light therapy.

The Nobel Prize for medicine was awarded to Finsen in 1903 for his activity in light therapy. He was the one who designed the first apparatus that technically generated beams similar to solar ones.

There are certain advantages in using the solar beams artificially generated such as: the parameters of intensity and the spectrum of the generated light are controllably so they are reproducible.

At about the end of the 1960's the German Neuropathologist Peter Mandel rediscovered the light therapy.

He said that every color of the spectrum is in strong connection with body's health: "the color penetrates the body' cells carrying with it the needed information for curing"

In the fact, that more than 40 inventions using light are registered in the whole world. These inventions are divided into three types:

### Laser type therapy

Inaugurated in 1967, it is characterized of using a certain wavelength and an energy transmitter, which is incorporated. Laser light is polar (vibrates in one single plan) and can be amplified at very high energies.

Even from that time it has been established that this type of light has a bio-stimulator effect on cells. So it means that tissues can be destroyed, we can effectuate very precise cuts and many other things depending on the laser type that we use.

### Bioptron type therapy

The spectrum of infrared and ultraviolet rays was the one that was used in modem light therapy at the beginning of the photo-medicine development.

In 1981 a team of Hungarian research- workers created a source of light that was initially based on laser therapy, of generating a combination of visible light and infrared radiation. The team considered light polarization as being an important parameter. The therapy system type Bioptron was created on this technology basis.

It is the most recent achievement that is used at international level right now, together with laser type therapy, having very good results and being also very well sold at a large scale.

It uses polychrome polarized light having wavelength and different colors, which has specific effects on cells. Polarization means that magnetic waves of light vibrate in parallel plans and

Built in many different types (different powers) it performs a superficial treatment on bodies especially at epidermis level.

*The Compact Bioptron* designed to be used at home, but also for special treatment in professional domains, too.

The apparatus consists of a special reflector with halogen bulb (20W) and a fan. It has a timer inside which emits a tone at an interval of 2 min.

*The Bioptron II* designed for professional use and it corresponds to international standards **TEC-601-1.** Bioptron II lamp is easy to handle. The case made of polyuretanical foam contents the whole optics of a reflector with halogen bulb (100W) and the timer and the electronic section, too. The treatment duration can be selected from 1 to 15 min. BIOPTRON AG Zepter has patented two invention:
a)Therapeutic lamp having
   - international publication WO 96/04958/22.02.1996 and
b)Therapeutic lamp irradiating polarized light, designed for manual use, having international publication no. WO 96/049508/22.02.1996 .

In the project for apparatus' description **ZEPTER** Company settled the method that makes possible the following activities:
- to influence the biologic system in a positive and commode manner
- to activate the capacity of self-defense
- to activate regenerating processes.

These apparatuses emit polarized light, which is emitted linearly on wavelengths between 400-9000nm.

The light waves cover the treated area on the visible side and the infrared spectrum. In this way cells can be treated till the depth of 2.5cm under the skin layer, without a significant heating of the tissues.

They do not contain ultraviolet rays and they have energy of 40nv/cm².

### I. HELIOSTROBOSCOP TYPE THERAPY

Conform with the international application WO 92/13597(20.08.1992) having no practical applicability, and left at experimental level.

The invention is defined in claim 1 below. The dependent claims are directed to optional features.

Our present invention is a medical apparatus with light flux, it belongs to **HELIOSTROBOSCOP** type and the present version has **PROBIOFOTON** title. It is designed as an original version, based on an electromagnetic field, emitted with a source of light, and it also has the purpose of making this way of treatment known at therapeutic level,

The present invention's purpose is to spread the application of biological treatments of irradiation with a light flux of determined-color.

The medical apparatus for biological treatments with a light flux it is used for modulating, separating and focusing a pencil of electromagnetic radiation emitted by a bulb with incandescence for obtaining energetic modifications in cellular biochemistry.

The medical apparatus resolves the problem of treating the non-congenital diseases with the aid of some bright radiation.

The technical problem that our present invention solves is to avoid the use of electric and magnetic fields in the light flux processing.

The medical apparatus, according to the invention, resolves the above problem because the electromagnetic field applied to a sick person it is obtained from a source of light put in the focus of a parabolic mirror.

In the light pencil's way, processed as we mentioned above, a rotating shutter and on objective are to be assembled on one of the case's inner sides, objective on which an optic filter is set.

A computer interface helps commanding and operating with the use of a program that controls the entire work of the apparatus.

The shutter disk has two elliptical orifices, which as a result of the variable-regulating device, design an ellipse with a little opening, less at one end, and circle-shaped at the end of the second ellipse.

The bright flux this way obtained is composed of direct rays from the light source and rays reflected from the parabolic mirror, focused through a lens.

The final pencil of rays this way obtained, after following through this optic labyrinth, has to be modulated at much lower frequencies comparing to the radiation that was used (higher wave lengths) for obtaining a long distance in penetrating the tissue.

The medical apparatus, according to the invention, has the following advantages:
- assures the certain obtaining of the necessary frequencies needed in treating some diseases
- now it is possible to use the treatment for people who have a pace-marker, and this is for the first time in applying this type of treatment using light
- this type of therapy can not only be combined with other types of treatment but can also fulfill their effect
- allows the co-ordination, depending on the case, of the naturist methods based on body detoxification and on introducing in diet some certain vitamins depending on the type of disease
- a very wide area of disease can be cured using it
- rapid and lasting results are obtained

The apparatus (1st picture), according to the invention, is made of a case 1 in which on a base plate 2 it is fixed a support 3 of the incandescent light type bulb 4.

For ensuring uniformity of ventilation for the bright item - bulb type 4, a fan 5 will be set in front of a main orifice **a.**

The interference between the incidental ray and the reflected ray, in coherent conditions, is obtained through one bulb operating.

The type of bulb 4 that we use - an incandescent one, has to be a pencil of light rays focused as much as possible on one point. The pencil's power is given by:
- the capacity of transmitting to distance
- the ray intensity
- the possibility of mechanic modulation, for avoiding the overheating effect on skin.

The power of bulb 4 with incandescence depends on the type of tissue that we irradiate. A gradual treatment of the sick person is recommended, proportional with the intensity of his/her disease at that time, till using the maximum intensity of the bulb 1500W, depending on case.

The source of light, bulb 4 type is set in the focus of a **parabolic mirror 6** made of a material with a high absorption coefficient for some frequency categories.

The mirror 6 positioning has to carry out the parallelism condition between the incidental ray and the reflected ray, having the possibility of displacing through the handle bar **b**.

The bulb 4 positioning to the reflecting item, mirror 6, has to carry out the conditions of obtaining some parallel light pencils.

For positioning the bulb 4 regarding the efficiency of the apparatus, in accordance with the gradual use of the its power, a gradual system **7** of bulb operating is recommended.

In the light pencil's way, processed as we mentioned above, a rotating shutter **8** is to be assembled on one of case's inner sides. This rotating shutter has two elliptical orifices **c** and **d** (1^{st} figure) which using a regulatory device for slits **9** (2^{nd} figure) design other different forms more efficient in treating.

The forms and dimensions c d on the rotating shutter 8 become variable depending on the frequency of the photons flux.

The shutter 8 opening slits with variable geometry figure the form of a small ellipse **c** at one end and the form of a small circle **d** at the other.

The ray processed on the shutter disc goes through an orifice **e** made on the inner side of the case and it is then directed through a lens 10 on which an optical filter 11 is fixed.

The shutter disk 8 is operated by an engine 12, which is directed by a digital voltage regulator 13 for obtaining the needed modulation frequencies (3^{rd} figure).

A fan 14 ventilates both the engine 12 and the digital voltage regulator.

The description and the scheme of the digital voltage regulator 13 in 64 stage displaying the engine's step 15 are the following:

It is a complex montage at the first sight having a relatively big number of chips, but at a closer sight you can see that every chip alone, is ordinary used, conforming to the dated in catalogue. Let's start with dividing the scheme in operating blocks:
A) Displaying block
B) Digital-analogic counter and decoding block
C) Command and control block
D) Power source

The first functioning block consists of two counters (MMC 40192), at whose outlets are connected to 7 segment / ach decoder / driver (MMC 4543), followed by two digits with common anode.

The second block consists of two counters (MMC 40193) from which the first six bits are selected, commanding six relays through a buffer with open-collector outlets. On the relays contacts there are parallel-assembled resistances, which during the countering make a variable resistance, depending on the pitch number, which varies the gate current of a triac through a diac.

Command and control block is made of the following circuits: MMC 4013 dual D-TYPE FLIP-FLOP; MMC 4048 (the logical programmable gate with eight inputs); MMC4060 (14 STAGE RIPPLE CARRY BINARY COUNTER / DIVIDER AND OSCILLATOR); two MMC circuits 4093 (four SI-NU logical gates (trigger Schmidt). The first bistable in MMC4013 chip receives the command "emergency stop" on the first 'reset' through a positive impulse from a reversible switch. The second command set on the same bistable is linked to the pin "clock" and execute the start-stop function. The bistable is linked in configuration of divider by two, it means that "data" pin is linked at the negative outlet. The outlets in fact represent the start/stop and inhibition physical commands, which are visually observed on a bicolor led, commanded by two bipolar transistors. When the led is red it means the montage is in "stand-by", when in fact the counters are initialized (MMC 40192 and MMC 40193) on the "preset enable" pin, charging the registers with the binary code 60. The meter of the divisor MMC 4060 is to be reset, and the MMC 4543 decoding-systems receive a command to turn the digits off and through a relay the engine's power is cut off. When a positive impulse shows up on the "clock" pin, the bistable moves back, the led will light green, and the MMC 40192 and MMC 40193 meters will receive the incrementation from the binary preset number, which is 60. Concomitantly, the MMC 4060 divisor will start to generate the clock signal for the above mentioned meters. The MMC 4543 diver will be enable to light the digits and the relay will be charged letting the voltage to power the engine. The next bistable of the MMC 4013 circuit controls the numbering direction, letting the clock signal (processed from MMC 4060) through two logical gates SI-NU, go to the incrementation or decrementation pins of the MMC 40192 and MMC 40193 meters. The "set" pin will receive a positive impulse through a condenser, from the "true" outlet of the other bistable, which will validate incrementaion when starting. Where "data" pin is, there is also the outlet of a gate SI-NU with two entries, which set the next numbering direction - depending on the "true" outlet of the bistable on the first entry, and on the second entry linked at the significant bit among those six that command the relays. This data will be charged when a positive impulse will appear at the "clock" pin, processed in the control block made of a MMC 4048 and a MMC 4093. This impulse appears in the moment when on the entries of the logical gate MMC 4048 there is the binary code "0" or "63", which means "000000" or "111111" that represent the end of the descremenation or incremenation, resulting from those six outlet bits of the MMC 40193 circuit. In any of those two situations, at the outlet "J" of the MMC 4048 gate, the logical "0" level appears. During incrementation, this gate will have the logical function SI-NU, and during decrementation- the logical function SAU-NU. Everything will be controlled at "Ka" pin linked to true outlet of the control bistable of direction. The outlet "J" of the logical gate MMC 4048 commands an astable with auto-reverse made of the four logical gates SI-NU having two entries each, of the second MMC 4093. When the 0 level appears in the entrance of the first gate, considering the fact that on the second gate is fixed the first logical level, the outlet would be in the logical level 1. The second gate receives on one gate the same fix level (logical 1) and n the other one the logical level 1 resulted from the first gate, the result on the outlet being the change into logical zero. There is a condenser between the outlet of the second gate and one of the entrances of the third one. This condenser is charged through two resistances 220k and 390k, through which it would discharge itself, when the logical level 0 appears from the outlet of the second gate. Until the condenser' discharging, the third gate will have at the outlet 0 logical level. The impulse will be inverted by the fourth gate, which has its entrances connected together, and transmitted to the "clock" pin of the control bistable of the clocking direction. This one will charge the logical level in "data" changing or not the metering direction. The last chip MMC 4060 produces the clocking signal visually indicated by the decimal point of the digit, which displays the decimal fractions. The oscillator frequency is varied by a potentiometer, so the signal received at the Q4 outlet will have it's own variable frequency, too. The metering signal could be a manual one (from a reversible switch) or an automatic one coming from the above-mentioned circuit, a commutable by a two position switch.

The power source is as simple as possible, made of: a transformer, a rectifying bridge, a filtering condenser and two stabilizing integrators of 5V and 12V, the first one powers the C-MOS circuits, it means the logical part; and the second charging the relays. Both circuits use the same heather sink.

This montage is integrated in the main functioning scheme of this apparatus, of transmitting a modulated light flux. In this way, the modulated flux once filtered through the shutter disk it is modulated in 64 degrees with the help of this digital modulator, for obtaining variable frequencies of the photons emitted by the source of light.

The flux modulation represents an element of novelty versus former technical achievements.

A computer 16 controls the apparatus' handling and coordinating.

For an efficient ventilation cycle, there were designed two ventilation slits f and g in the back side of the a apparatus, concordant with the ventilation made by (5-14) fans, both in the superior side and near the foot plate.

The final pencil resulted after going through this optic labyrinth has to have a minimum intensity, to be mechanic modulated at much lower frequencies comparing with the used radiation (longer wavelengths). Reviewing the constitutive elements of this **MEDICAL**

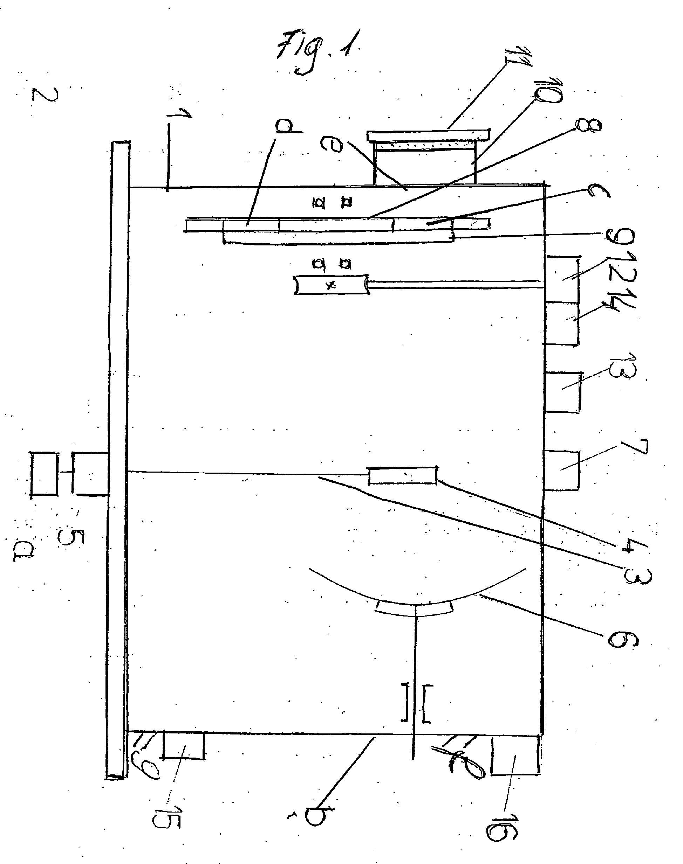

### APPARATUS WITH LIGHT PENCIL (figure 1) we remind the following:

**I. MAIN ELEMENTS**
   1) case
   2) foot plate
   3) bulb support
   4) incandescent bulb
   5) bulb fan
   6) parabolic mirror
   7) gradual system for lightning the bulb
   8) coring shutter
   9) slits regulating device
   10) apparatus' object
   11) optic filter
   12) rotating disc's engine
   13) digital modulator of the light flux
   14) engine's fan
   15) engine's pitch display
   16) computer
**II. ADDITIONAL ACCESSORIES**
   a) main orifice in ventilation
   b) handle bar for the mirror
   c) shutter disc' orifice with slit for handling
   d) objective orifice
   e) case's ventilating slit
**III. OTHER DIFFERENT CONSTITUTING ELEMENTS**
   Fig. 1 The ensemble scheme of the apparatus

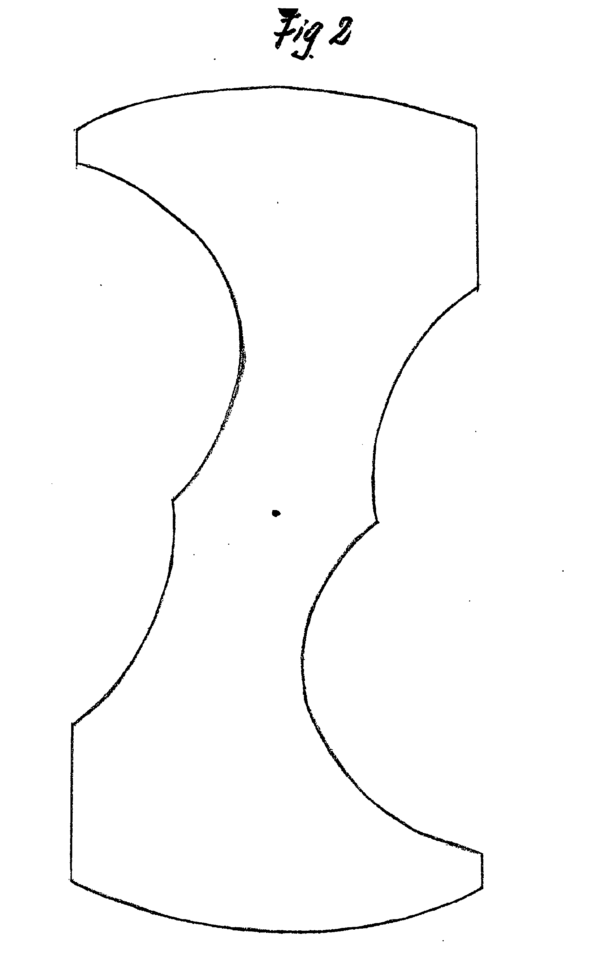

   Fig. 2 Slits regulating device

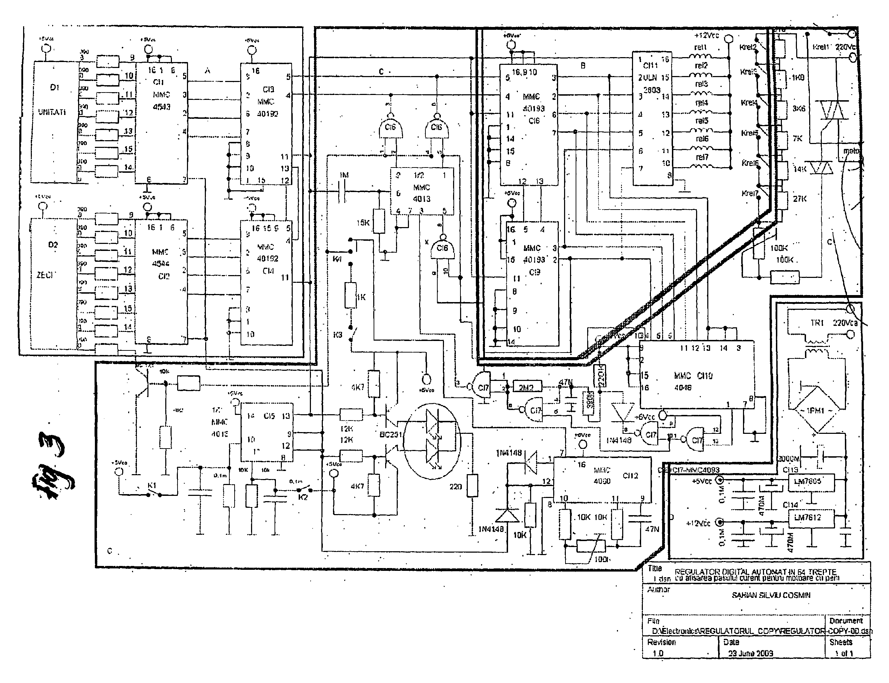

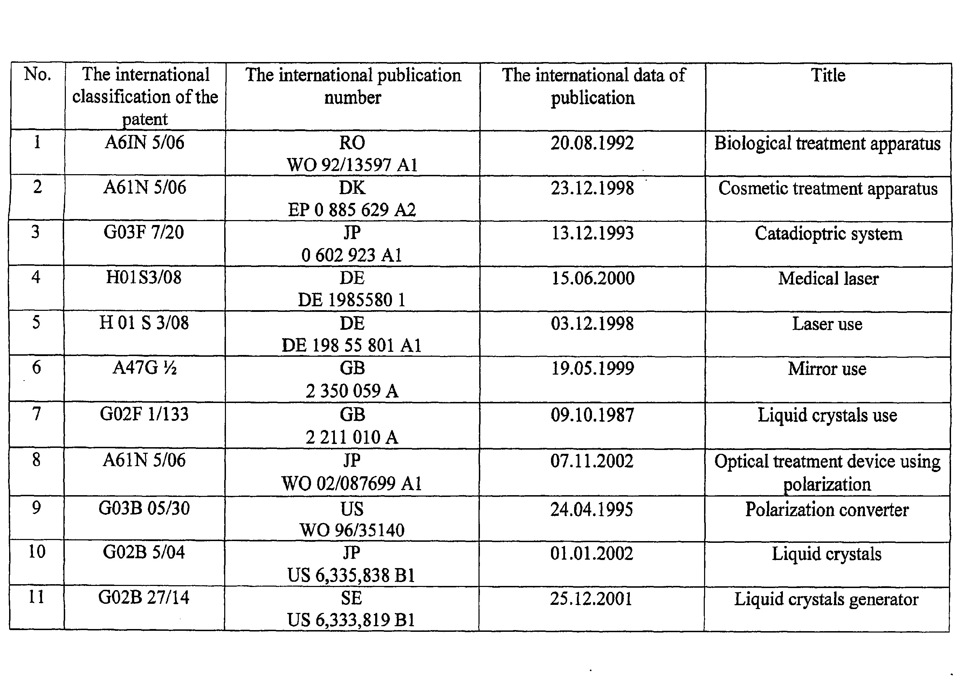

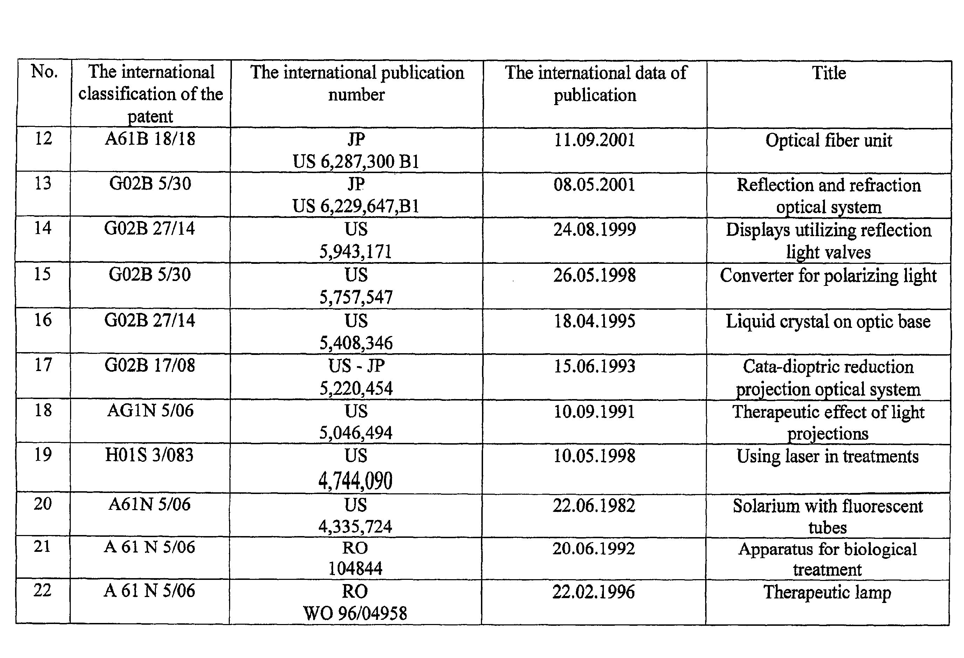

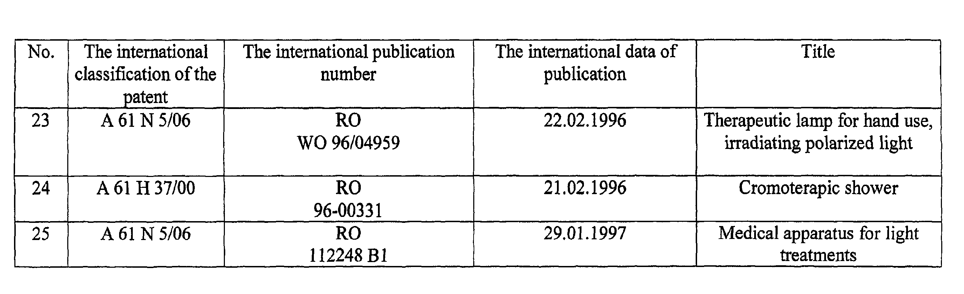

   Fig. 3 The electronic scheme of the light flux' digital modulator
   Fig. 4 Elliptical slits' disc
**IV. TABLE OF INVENTIONS THAT USE THE LIGHT FLUX IN THERAPY**
   (annex IV)
**V. EXCERPTS FROM PATENTS THAT USE LIGHT IN THERAPY**
   (annex V)

The most representative inventions at international level, which are opposed to our invention are patented as follows:
1. International Application published WO 92/13597-20.08.1992 inventor Carol Przybilla
2. International Application published WO 96/04958-22.02.1996 applicant BIOPTRON AG-MONCHALTOR F (CH)

The elements that make us different from all these are:

### I. The shutter disc 8 performances

As a result of our studies, we ascertained that the shutter disc dimension of 150 mm, according to application WO92/13597 (1992), should be changed to the optimum dimension of 220 mm.

The elliptical orifices c and d of the shutter disk 8 dimension it's proportional with the light flux intensity and concords with the rotating frequency of the disc. Using this way calculated shutter disc 8, the frequency and amplitude modulation of the electromagnetic field is possible.

### II. Regulating slits device 9

Associated with the dimension of the shutter disc 8, makes the two ellipses have different forms and dimensions, in contrast with WO 92/13597. If in this application, the ratio of the ellipses' axis of the orifices (cd) is 066-095, in our description for patenting, the form and the dimension of cd slits on the shutter disc 8 are variable, depending on the photons' flux frequencies, which are imposed by the sick people' diagnosis.

Due to the slits regulating device 9, the orifice of the slit c designs the form of a small ellipsis, and the orifice of the slit d designs the form of a small circle

In WO 92/13597 application, the slits which are very big have the disadvantage of a weaker control for obtaining the modulation frequencies.

### III. The digital modulator of the light flux 13

After many years of studying we have reached the conclusion that also the system of varying the frequencies with the help of the digital modulator 13, with the current pitch of the engine display 15, is concordant with the energetic centers of different types of tissue treating, subdue to cells' modifications.

The rotating frequency of the shutter disc 8 is variable for encountering the area of necessary requirements in treating the energetic centers of many different types of tissue.

The area of speed for the shutter disc is between 1550-3000 turn/min., opposed to WO 92/13597 application where the frequency variation is inexistent.

Both the obtaining of the variation of frequencies and that of a certain types of photons in the electromagnetic field that is used, doesn't depend on the nurse's medical training in using the apparatus, since it is an automatic system.

### IV. The bulb power (4) supplier of the electromagnetic field

Higher from 600-1000W in WO 92/13597 patent, to 1500W.

It is another important item that makes us different from all the others opposable patents mentioned above.

The length of the irradiating flux at this level it's another innovation which makes the described energetic treatment more efficient.

It is enough to remind about the Bioptron type apparatus used today, where the maximum power of the light when functioning can not be over 100W.

**V. Electromagnetic frequency band used when referring to** this type of apparatus is between 560-3000nm, a frequency obtained through the variation of the functioning parameters of the computer's command and regulation systems.

The variation of the electromagnetic frequencies can be also obtained if varying the power of the incandescent bulb, being electronically controlled and the power area being set in the computer's command program.

### VI. The engine's pitch display

It is the most interesting innovation because allows us a permanent control of the apparatus' functioning parameters when doing the treatment.

### VII. Computer's (16) help in coordination

Through its functioning, the computer assures the command and control for all variable items inside the system, the ability of the stuff that supervises it being not necessary for this activity.

### VIII. Turning cylinders (12) in WO 92/13597 application

They were eliminated because they were practically inefficient for obtaining a beating phenomenon during the modulation and interference process of the light pencil.

### IX. The apparatus dimension is obvious superior to those two types of opposable apparatuses described above, and it is imposed by:

- the shutter disc 8 dimension
- the engine 12 placement
- the incandescent bulb's power 14
- the parabolic mirror 6 dimensions
- the ventilation and fanning system (5-14 f, g)
- computer's placement 16

While the opposable patents were made for an ambulatory treatment - being portable, the functioning of this apparatus and the method of treatment require a special room, because its power can lead to other diseases or adverse reactions, that need a closer look from the doctors.

### X. The non-noxious treatment is valid if the technically conceived parameters and their functioning are respected.

Due to the certain wavelengths that we use, we can cure sick persons who:
- bear a pace-makers
- bear metallic rods

Due to our experience, sick persons that could follow this treatment could be between 1-80 years old; the optimum results being obtained when the ideal age is between 1-60 years old if the human cell is receptive to the modifications imposed by the apparatus.

### XII. The parabolic mirror (6), made of a material with great absorption coefficient of some frequencies' categories is specially designed.

### XIII. This is a complementary treatment to allopathic and naturist medicine that offers a substantial support in obtaining the best results.

At the body's level: cell's reorganization, organ's function normalizing, accelerating and maintaining the health condition.

The invention has the following advantages:
- the secure obtaining of the necessary frequencies needed in treating some diseases
- it doesn't use electromagnetic damaging fields for health
- easy to use
- it is possible to use the treatment for sick people bearing a pace
- maker

## Claims

1. A medical apparatus for producing light flux for biological treatments, the apparatus comprising: a case (1), a support (3) which bears a light source bulb (4), a concave mirror (6) set on an axial bar on one wall of the case (1), wherein on the opposite wall there is an optical filter (11) mounted on an objective (10) in an orifice of the case (1) and wherein a rotating shutter disc (8) comprising orifices (c, d) is mounted on the same wall as the optical filter (11) in such a way, that the orifices (c, d) become coaxial with the optical filter axis during rotation of the shutter disc (8) the light flux being made up of the rays reflected by the concave mirror (6) and of the direct rays emitted by the light source bulb (4), the rays passing through the orifices (c, d) of the rotating shutter disc (8) to the objective (10) **characterised in that** the rotating shuffer disc (8) is equipped with a slit regulating device (9).

2. The medical apparatus according to claim 1, wherein the shutter disc (8) is rotated by an engine (12), ventilated by a fan (14) and controlled by a digital voltage modulator (13).

3. The medical apparatus according to claim 2, wherein a computer (16) controls the entire apparatus' handling and coordinating.

4. The medical apparatus according to claim 1, wherein the support (3) is set on a footplate (2).

5. The medical apparatus according to any one of preceding claims 1, 2 or 4, wherein the light source bulb (4) is ventilated by a fan (5) mounted inside the case (1).

6. Medical apparatus according to claim 1, **characterized in that** the size of the rotating shutter disk (8) orifices is proportional to the light flux intensity and concords with the rotational frequency of the rotating shutter disc (8).

7. Medical apparatus according to claim 1, **characterized in that** the electromagnetic frequency band of the light source bulb (4) is between 560 and 3000 nm.

8. Medical apparatus according to claim 1, **characterized in that** the concave mirror (6) is a parabolic mirror in the focus of which the light source bulb (4) is mounted.

## Patentansprüche

1. Medizinisches Gerät zur Erzeugung eines Lichtstroms für biologische Behandlungen, wobei das Gerät ein Gehäuse (1), eine Halterung (3), die eine Lichtquelle (4) stützt, einen konkaven Spiegel (6), der an den Axialstab einer der Gehäusewände befestigt ist (1), umfasst, wobei auf der gegenüber liegenden Gehäusewand sich ein optischer Filter (11) befindet, der in einer Gehäuseöffnung (1) auf das Objektiv (10) montiert ist, und an derselben Wand wie der optischen Filter (11) eine drehbare Blendenscheibe (8) montiert ist, die Öffnungen (c, d) aufweist, so dass während der Drehung der Blendenscheibe die Öffnungen (c, d) koaxial zur Achse des optischen Filters werden, wobei der Lichtstrom aus den von dem konkaven Spiegel (6) widerspiegelten Strahlen und den von der Lichtquelle (4) abgegebenen direkten Strahlen besteht, wobei die Strahlen durch die Öffnungen (c, d) der drehbaren Blendenscheibe (8) auf das Objektiv (10) zulaufen, **dadurch gekennzeichnet, dass** die drehbare Blendenscheibe (8) mit einer Vorrichtung (9) zur Regelung des Schlitzes ausgestattet ist.

2. Medizinisches Gerät nach Anspruch 1, wobei die Blendenscheibe (8) von einem Motor (12) gedreht, mit einem Ventilator (14) belüftet und durch einen digitalen Spannungsmodulator (13) kontrolliert wird.

3. Medizinisches Gerät nach Anspruch 2, wobei ein Computer (16) die vollständige Bedienung und Synchronisation des Gerätes kontrolliert.

4. Medizinisches Gerät nach Anspruch 1, wobei die Halterung (3) auf einer Halteplatte (2) befestigt ist.

5. Medizinisches Gerät nach einem der Ansprüche 1, 2 oder 4, wobei die Lichtquelle (4) durch einen Ventilator (5) belüftet wird, der innerhalb des Gehäuses (1) montiert ist.

6. Medizinisches Gerät nach Anspruch 1, **dadurch gekennzeichnet, dass** die Abmessung der Öffnungen der drehbaren Blendenscheibe (8) proportional zu der Intensität des Lichtstroms ist und mit der Drehfrequenz der drehbaren Blendenscheibe (8) übereinstimmt.

7. Medizinisches Gerät nach Anspruch 1, **dadurch gekennzeichnet, dass** das elektromagnetischen Frequenzband der Lichtquelle (4) zwischen 560 und 3000 nm liegt.

8. Medizinisches Gerät nach Anspruch 1, **dadurch gekennzeichnet, dass** es sich bei dem konkaven Spiegel (6) um einen Parabolspiegel handelt, in dessen Brennpunkt die Lichtquelle (4) montiert ist.

## Revendications

1. Un appareil médical pour produire un flux lumineux pour des traitements biologiques, l'appareil contenant: une carcasse (1), un support (3) qui soutient une source de lumière (4), un miroir (6) concave assis sur la barre axiale d'un des murs de la carcasse (1), où sur le mur opposé se trouve un filtre (11) optique monté sur l'objectif (10) dans un orifice de la carcasse (1) et un disque (8) obturateur rotatif contenant les orifices (c, d) est monté sur le même mur comme le filtre (11) optique, de manière que les orifices (c, d) deviennent coaxiales avec l'axe du filtre optique, au cours de la rotation du disque (8) obturateur, le flux de lumière étant formé des rayons reflétés par le miroir (6) concave et les rayons directs émis par la source de lumière (4), les rayons passant par les orifices (c, d) du disque (8) obturateur rotatif vers l'objectif (10) **caractérisé par le fait que** le disque (8) obturateur rotatif est prévu d'un dispositif (9) de réglage de la fente.

2. L'appareil médical conforme à la revendication 1, où le disque (8) obturateur est tourné par un moteur (12), ventilé par un ventilateur (14) et contrôlé par un modulateur de tension digital (13).

3. L'appareil médical conforme à la revendication 2, où un ordinateur (16) contrôle l'ensemble des manoeuvres et activités de synchronisation de l'appareil.

4. L'appareil médical conforme à la revendication 1, où le support (3) est assis sur une plaque de soutien (2).

5. L'appareil médical conforme à n'importe quelle des revendications précédentes 1, 2 ou 4 où la source de lumière (4) est ventilée par un ventilateur (5) monté à l'intérieur de la carcasse (1).

6. Appareil médical conforme à la revendication 1, **caractérisé par le fait que** la dimension des orifices du disque (8) obturateur rotatif est proportionnelle à l'intensité du flux lumineux et en accord à la fréquence de rotation du disque (8) obturateur rotatif.

7. Appareil médical conforme à la revendication 1, **caractérisé par le fait que** la bande de la fréquence électromagnétique de la source de lumière (4) est entre 560 et 3000 nm.

8. Appareil médical conforme à la revendication 1, **caractérisé par le fait que** le miroir (6) concave est un miroir parabolique, dans le foyer duquel est montée la source de lumière (4).
